# EUROPÄISCHE PATENTSCHRIFT

(11) **EP 1 123 409 B1**
(45) Veröffentlichungstag und Bekanntmachung des Hinweises auf die Patenterteilung: **07.06.2006**
(21) Anmeldenummer: 99953891.1
(22) Anmeldetag: 20.10.1999
(51) Int. Cl.: C12P 17/04, C12P 7/62, C12P 7/42, C12N 1/16, C12R 1/645, C12R 1/73

(54) **VERFAHREN ZUR GEWINNUNG VON GAMMA-DECALACTON**
METHOD OF PRODUCING GAMMA-DECALACTONE
PROCEDE DE PREPARATION DE GAMMA-DECALACTONE

(30) Priorität: 24.10.1998 EP 98120206
(43) Veröffentlichungstag der Anmeldung: 16.08.2001
(73) Patentinhaber: Symrise GmbH & Co. KG, 37603 Holzminden (DE)
(72) Erfinder: RABENHORST, Jürgen, D-37671 Höxter (DE); GATFIELD, Ian, D-37671 Höxter (DE)
(74) Vertreter: Eisenführ, Speiser & Partner
(86) Internationale Anmeldenummer: PCT/EP1999/007950
(87) Internationale Veröffentlichungsnummer: WO 2000/024920

(56) Entgegenhaltungen:
- DE-A- 4 126 997
- FR-A- 2 734 843
- CHEMICAL ABSTRACTS, vol. 122, no. 21, 22. Mai 1995 (1995-05-22) Columbus, Ohio, US; abstract no. 263911, GATFIELD, I. L. ET AL: "The enzymic and fermentative production of lactones and their use in natural flavors" XP002105362 & RECENT DEV. FLAVOR FRAGRANCE CHEM., PROC. INT. HAARMANN REIMER SYMP., 3RD (1993), 291-304. EDITOR(S): HOPP, RUDOLF;MORI, KENJI. PUBLISHER: VCH, WEINHEIM, GERMANY. CODEN: 60ZGAH,
- CHEMICAL ABSTRACTS, vol. 121, no. 19, 7. November 1994 (1994-11-07) Columbus, Ohio, US; abstract no. 229299, GATFIELD, I. L. ET AL: "Aspects of the microbiological manufacture of flavor-active lactones with particular reference to. gamma.- decalactone" XP002105363 & CHEM., MIKROBIOL., TECHNOL. LEBENSM. (1993), 15(5/6), 165-70 CODEN: CMTLBX;ISSN: 0366-7154,
- CHEMICAL ABSTRACTS, vol. 127, no. 17, 27. Oktober 1997 (1997-10-27) Columbus, Ohio, US; abstract no. 233574, PAGOT, Y. ET AL: "Utilization of an auxotrophic strain of the yeast Yarrowia lipolytica to improve. gamma.- decalactone production yields" XP002105364 & LETT. APPL. MICROBIOL. (1997), 25(2), 113-116 CODEN: LAMIE7;ISSN: 0266-8254,
- CHEMICAL ABSTRACTS, vol. 121, no. 25, 19. Dezember 1994 (1994-12-19) Columbus, Ohio, US; abstract no. 295696, MORGUNOV, I. G. ET AL: "Isolation, purification and some properties of citrate synthase from citric acid-accumulating yeast Yarrowia ( Candida ) lipolytica" XP002105365 & BIOKHIMIYA (MOSCOW) (1994), 59(9), 1320-9 CODEN: BIOHAO;ISSN: 0320-9725,

## Beschreibung

Die vorliegende Erfindung betrifft ein neues Verfahren zur Herstellung von γ-Decalacton.

γ-Decalacton ist aufgrund seiner organoleptischen Eigenschaften ein wichtiger Aromastoff, der einen fruchtigen, pfirsichartigen Geschmack und Geruch aufweist. Grundsätzlich kann γ-Decalacton aus Früchten gewonnen werden. In diesen ist er jedoch in so geringen Mengen vorhanden, dass er wirtschaftlich mittels Extraktion oder Destillation sich nicht isolieren läßt.

Deshalb gab es in den letzten Jahren zahlreiche Versuche, γ-Decalacton in biotechnischen Verfahren herzustellen. Die meisten Verfahren arbeiten unter Einsatz von verschiedenen Hefen. Hierbei werden entweder Rizinusöl oder der Methylester der daraus isolierten Rizinolsäure mit den Hefen umgesetzt. Die dabei erzielten Ausbeuten schwanken zwischen wenigen Milligramm pro Liter bis zu 9,4 g/l in 75 Stunden (FR 2 734 843).

Das Verfahren hat außerdem den Nachteil, dass mit einem Uracil-auxotrophen Material gearbeitet wird und damit zwei getrennte Schritte zur Biomassebildung und Produktion notwendig sind.

Folgende Dokumente beschreiben weitere Verfahren zum Herstellen von γ-Decalacton unter Verwendung von *Candida lipolytica* bzw. *Yarrowia lipolytica:*

Chemical Abstracts, Vol. 122, No. 21, Nr. 263911; Chemical Abstracts, Vol. 121, No. 19, Nr. 229299; Chemical Abstracts, Vol. 127, No. 17, Nr. 233574; DE 41 26 997 A. Darüber hinaus ist Chemical Abstracts Vol. 121, No. 25, Nr. 295696 zu entnehmen, dass *Candida lipolytica* und *Yarrowia lipolytica* identisch sind.

Aufgabe der vorliegenden Erfindung ist es daher, ein Verfahren zur Verfügung zu stellen, das das Erzielen höherer Ausbeuten ermöglicht.

Die Aufgabe wird gelöst durch ein Verfahren zur Herstellung von γ-Decalacton, umfassend die Schritte:
a) Auswählen eines Stammes von *Yarrowia lipolytica,* der in weniger als 70 Stunden mehr als 11 g/l γ-Decalacton erzeugen kann, und
b) in einem Substrat Kultivieren des in a) ausgewählten Stammes zum Herstellen von γ-Decalacton.

Es ist erfindungsgemäß möglich, Yarrowia lipolytica im Gemisch mit weiteren Mikroorganismen zu verwenden. Vorzugsweise wird Yarrowia lipolytica jedoch als Reinkultur eingesetzt. Besonders bevorzugt ist es erfindungsgemäß, den Stamm Yarrowia lipolytica HR145 (DSM 12397) zu kultivieren.

Als Substrat für die erfindungsgemäß eingesetzte Kultur kommen synthetische, halb-synthetische oder komplexe Kulturmedien in Betracht. Diese enthalten kohlenstoffhaltige und stickstoffhaltige Verbindungen, anorganische Salze, gegebenenfalls Spurenelemente sowie Vitamine.

Als kohlenstoffhaltige Verbindungen kommen vorzugsweise Kohlenhydrate, Kohlenwasserstoffe oder organische Grundchemikalien in Betracht. Beispiele für vorzugsweise verwendbare Verbindungen sind Zucker, Alkohole bzw. Zuckeralkohole, organische Säuren oder komplexe Gemische. Bevorzugt sind erfindungsgemäß Öle.

Als Zucker kommt vorzugsweise Glucose in Betracht. Zu den verwendbaren Alkoholen gehören vorzugsweise Glycerin oder Mannit. Als organische Säuren kann vorzugsweise Zitronensäure zum Einsatz kommen. Zu den komplexen Gemischen zählen z.B. Malzextrakt, Hefeextrakt, Casein oder Caseinhydrolysat. Als Öl ist insbesondere Rizinusöl verwendbar. Hierbei können erfindungsgemäß auch Gemische zweier oder mehrerer der genannten Verbindungen eingesetzt werden.

Als stickstoffhaltige Substrate kommen anorganische Verbindungen in Betracht. Beispiele hierfür sind Nitrate und Ammoniumsalze. Ebenso können organische Stickstoffquellen zum Einsatz kommen. Hierzu zählen Hefeextrakt, Sojamehl, Baumwollsaatmehl, Casein, Caseinhydrolysat, Weizengluten und Maisquellwasser. Möglich ist auch der Einsatz zweier oder mehrerer der genannten Verbindungen als Gemisch.

Zu den einsetzbaren anorganischen Salze zählen beispielsweise Borate, Carbonate, Chloride, Molybdate, Nitrate, Phosphate und Sulfate. Als Metalle enthalten die genannten Salze vorzugsweise Calcium, Eisen, Kalium, Kobalt, Kupfer, Magnesium, Mangan, Natrium und Zink. Erfindungsgemäß kann auch ein Gemisch zweier oder mehrerer der genannten Salze verwendet werden.

Die Temperatur für die Kultivierung liegt vorzugsweise im Bereich von 10 bis 40°C. Besonders bevorzugt ist der Bereich von 20 bis 35°C, höchst bevorzugt sind 25 bis 30°C.

Der pH-Wert des Mediums beträgt bevorzugt 4 bis 9. Besonders bevorzugt ist der Bereich von 5 bis 8.

Während des Herstellungsverfahrens ist eine ausreichende Belüftung erforderlich. Entsprechend sind die erfindungsgemäß einsetzbaren Reaktoren auszulegen. Grundsätzlich können somit erfindungsgemäß alle dem Fachmann bekannten Bioreaktoren eingesetzt werden, die für aerobe Verfahrensweisen geeignet sind. Vorzugsweise kommen alle für alle aerobe Submersverfahren geeigneten Vorrichtungen Betracht. Das heißt, es können erfindungsgemäß Gefäße ohne oder mit mechanischer Mischeinrichtung eingesetzt werden. Zu ersteren zählen z B. Schüttelapparaturen, Blasensäulen- oder Schlaufenreaktoren. Zu letzteren gehören vorzugsweise alle bekannten Vorrichtungen mit Rührem in beliebiger Gestaltung.

Das erfindungsgemäße Verfahren kann kontinuierlich oder diskontinuierlich durchgeführt werden. Die Dauer der Fermentation bis zum Erreichen einer maximalen Produktmenge liegt im Bereich von 36 und 72 Stunden, vorzugsweise im Bereich von 48 und 66 Stunden, gerechnet von dem Animpfen der Kultur.

Erfindungsgemäß können die Substrate zu Beginn der Inkubation, während oder nach Abschluß des Wachstums zugegeben werden. Dies kann mit einer einmaligen Zugabe an Substraten oder durch kontinuierliche, sukzessive Zugabe während des Prozesses erfolgen.

Bevorzugt ist jedoch die kontinuierliche Zugabe über einen Zeitraum von mehreren Stunden nach dem Animpfen der Kultur.

Mit den beschriebenen erfindungsgemäßen Verfahren ist es überraschenderweise möglich, in weniger als 70 Stunden mehr als 11 g/l γ-Decalacton zu erzeugen. Zu den folgenden Beispielen werden die Erfindung und die damit verbundenen Überraschungen näher erläutert.

### Beispiele

### 1. Herstellung der Vorkultur

1,7 g Malzextrakt werden in 100 ml Wasser in einem 500 ml Erlenmeyerkolben mit seitlichem Einstich gelöst und für 15 Minuten bei 121 °C im Autoklaven sterilisiert. Nach dem Abkühlen auf Raumtemperatur wird der Malzbouillon-Kolben mit einer Impfschlinge von einer Schrägröhrchenkultur von Yarrowia lipolytica HR 145 angeimpft. Der Kolben wird für 24 Stunden auf der rotierenden Schüttelmaschine bei 27°C und 100 Upm inkubiert.

Zwei 500 ml Erlenmeyerkolben mit seitlichem Einstich werden mit Medium (1,461 g Na₂HPO₄ x 12 H₂O; 0,352 g KH₂PO₄; 0, 53 g Harnstoff; 0,07 g Tween 80; 5 g Hefepulver; 1 g Rizinusöl und 100 ml Wasser) beschickt und für 15 Minuten bei 121 °C im Autoklaven sterilisiert. Nach Abkühlen auf Raumtemperatur werden die Kolben mit je 500 µl einer Malzextrakt-Bouillonkultur von Yarrowia lipolytica HR 145 angeimpft.

Die Kolben werden für 24 Stunden auf der rotierenden Schüttelmaschine bei 27°C und 100 Upm inkubiert.

### 2. Produktion von γ-Decalacton im 10 l Fermenter

Es werden 9,8 1 Wasser im Fermenter vorgelegt und 14,61 g Na₂HPO₄ x 12 H₂O, 53 g Harnstoff , 50 g MgSO₄ x 7 H₂O, 0,04 g Riboflavin, 500 g Hefepulver, 7,0 g Tween 80, 100 g Rizinusöl und 5 g Antischaummittel zugefügt. Das Medium wird in situ 30 Min. bei 121°C sterilisiert. Weiterhin werden je 500 g verdünnte Natronlauge und Schwefelsäure sowie Rizinusöl im Autoklaven sterilisiert. Nach dem Abkühlen werden die Antischaumsonde und die Anstechgarnitur für NaOH angeschlossen. Der pH-Wert beträgt nach der Sterilisation etwa pH 7,9. Mittels der verdünnten Schwefelsäure wird ein pH-Wert von pH 7,0 eingestellt. Die Drehzahl der Rührung beträgt 400 Upm; die Belüftung 3 1/min Druckluft; die Temperatur 27°C.

Der Fermenter wird über eine sterile Anstechgarnitur mit der Vorkultur angeimpft. Während der Fermentation wird weitere Natronlauge zudosiert, um den pH-Wert bei pH 7,0 zu halten. Bei Bedarf wird automatisch Entschäumer zudosiert. 14 Stunden nach dem Animpfen wird mit der Substratzugabe begonnen. Innerhalb von 4 Stunden werden 500 g Rizinusöl zugegeben.

Nach einer Fermentationszeit von ca. 52 Stunden wird die Fermentation beendet. Der Zeitpunkt für den Abbruch der Fermentation ist erreicht, wenn eine Stunde keine weitere Natronlauge zudosiert wurde. Um die Fermentation abzubrechen, wird der Fermenterinhalt mit konzentrierter Schwefelsäure auf pH 2,0 eingestellt und 30 Min. auf 80°C erhitzt. Nach dem Abkühlen wird er für die nachfolgende Aufarbeitung abgefüllt. Die Endkonzentration des γ-Decalactons beträgt laut HPLC 11.500 bis 12.500 ppm. Das Verhältnis 3-Hydroxy-γ-Decalacton zu γ-Decalacton ist kleiner 0,2.

Aus der so erhaltenen Kulturbrühe wird dann, gegebenenfalls nach Zentrifugation, das γ-Decalacton mit den bekannten physikalischen Verfahren (Destillation, Extraktion, etc.) isoliert.

### 3. Produktion im 300 l Maßstab

Es wurden 200 ml Vorkultur genau nach Beispiel 1 hergestellt und zum Beimpfen eines 300 l Fermenters verwendet. Zuvor wurden 140 l Wasser im Fermenter vorgelegt und 742 g Harnstoff, 49 g KH₂PO₄, 12 g H₂O, 7 g Hefeextrakt, sowie 98 g Tween 80 hinzugefügt. Das Medium wird in situ 30 Minuten bei 121°C sterilisiert.

Nach dem Abkühlen werden die Antischaumsonde und die Anstechgarnitur für NaOH angeschlossen. Der pH-Wert beträgt nach der Sterilisation etwa 7,0. Die Drehzahl des Rührers wird auf 180 Upm, die Belüftung auf 35 l/min und die Temperatur auf 27°C eingestellt. Der Fermenter wird unter sterilen Bedingungen mit den 200 ml Vorkultur angeimpft. Während der Fermentation wird mittels Natronlauge der pH-Wert auf pH 7,0 konstant gehalten. Etwa 16 Stunden nach dem Animpfen wird mit der Rizinusölzugabe (7,4 kg) begonnen, welche nach weiteren 4 Stunden abgeschlossen ist.

Nach einer Fermentationszeit von 69 Stunden wird die Fermentation beendet. Die Endkonzentration des γ-Decalactons beträgt 12,3 g/l. Das Verhältnis 3-Hydroxy-γ-Decalacton zu γ-Decalacton beträgt 0,125, da nur 1,53 g/l 3-Hydroxy-γ-Decalacton gebildet werden.

## Patentansprüche

1. Verfahren zur Herstellung von γ-Decalacton, umfassend die Schritte:
a) Auswählen eines Stammes von *Yarrowia lipolytica,* der in weniger als 70 Stunden mehr als 11 g/l γ-Decalacton erzeugen kann, und
b) in einem Substrat Kultivieren des in a) ausgewählten Stammes zum Herstellen von γ-Decalacton.

2. Verfahren nach Anspruch 1, **dadurch gekennzeichnet, dass** in Schritt b) eine Reinkultur des in Schritt a) ausgewählten Stammes kultiviert wird.

3. Verfahren nach einem der Ansprüche 1 bis 2, **dadurch gekennzeichnet, dass** in Schritt a) *Yarrowia lipolytica* HR 145 (DSM 12397) ausgewählt wird.

4. Verfahren nach einem der Ansprüche 1 bis 3, **dadurch gekennzeichnet, dass** zum Kultivieren in Schritt b) ein synthetisches, halbsynthetisches oder komplexes Substrat verwendet wird.

5. Verfahren nach einem der Ansprüche 1 bis 4, **dadurch gekennzeichnet, dass** zum Kultivieren in Schritt b) kohlenstoffhaltige sowie stickstoffhaltige Verbindungen, anorganische Salze, Spurenelemente und/oder Vitamine enthaltende Substrate verwendet werden.

6. Verfahren nach Anspruch 5, **dadurch gekennzeichnet, dass** als kohlenstoffhaltige Verbindungen Zucker, Zuckeralkohole, Alkohol, organische Säuren, komplexe Gemische, Öle oder Gemische zweier oder mehrerer dieser Substanzen verwendet werden.

7. Verfahren nach Anspruch 6, **dadurch gekennzeichnet, dass** in Schritt b) ein Substrat enthaltend Glucose, Glyzerin, Mannit, Zitronensäure, Malzextrakt, Hefeextrakt, Casein, Caseinhydrolysat und Rizinusöl oder Gemische zweier oder mehrerer dieser Substanzen verwendet wird.

8. Verfahren nach einem der Ansprüche 1 bis 7, **dadurch gekennzeichnet, dass** in Schritt b) ein Substrat enthaltend anorganische Verbindungen und/oder organische Verbindungen verwendet wird.

9. Verfahren nach Anspruch 8, **dadurch gekennzeichnet, dass** in Schritt b) ein Substrat enthaltend Nitrate, Ammoniumsalze, Hefeextrakt, Sojamehl, Baumwollsaatmehl, Casein, Caseinhydrolysat, Weizengluten und Maisquellwasser verwendet wird.

10. Verfahren nach einem der Ansprüche 1 bis 9, **dadurch gekennzeichnet, dass** in Schritt b) ein Substrat enthaltend Sulfate, Nitrate, Chloride, Carbonate und Phosphate der Metalle Natrium, Kalium, Magnesium, Mangan, Calcium, Zink und Eisen oder Gemische zweier oder mehrerer dieser Verbindungen verwendet wird.

11. Verfahren nach einem der Ansprüche 1 bis 10, **dadurch gekennzeichnet, dass** Schritt b) bei einer Temperatur im Bereich von 10 bis 40°C durchgeführt wird.

12. Verfahren nach einem der Ansprüche 1 bis 11 **dadurch gekennzeichnet, dass** Schritt b) bei einem pH-Wert im Bereich von 4 bis 11 durchgeführt wird.

13. *Yarrowia lipolytica* HR 145 (DSM 12397).

14. Verwendung von *Yarrowia lipolytica* HR 145 (DSM 12397) zum Herstellen von γ-Decalacton.

## Claims

1. A process for the production of γ-decalactone, comprising the following steps:
a) selecting a strain of *Yarrowia lipolytica* that can produce more than 11 g/l of γ-decalactone in less than 70 hours, and
b) cultivating the strain selected in a) in a substrate for the production of γ-decalactone.

2. The process according to claim 1, **characterised in that**, in step b), a pure culture of the strain selected in step a) is cultivated.

3. The process according to one of claims 1 to 2, **characterised in that**, in step a), *Yarrowia lipolytica* HR 145 (DSM 12397) is selected.

4. The process according to one of claims 1 to 3, **characterised in that**, in step b), a synthetic, semisynthetic or complex substrate is used for the cultivation.

5. The process according to one of claims 1 to 4, **characterised in that**, in step b), substrates containing carbon compounds and nitrogen compounds, inorganic salts, trace elements and/or vitamins are used for the cultivation.

6. The process according to claim 5, **characterised in that** sugars, sugar alcohols, alcohol, organic acids, complex mixtures, oils or mixtures of two or more of these substances are used as carbon compounds.

7. The process according to claim 6, **characterised in that**, in step b), a substrate containing glucose, glycerol, mannitol, citric acid, malt extract, yeast extract, casein, casein hydrolysate and castor oil or mixtures of two or more of these substances is used.

8. The process according to one of claims 1 to 7, **characterised in that**, in step b), a substrate containing inorganic compounds and/or organic compounds is used.

9. The process according to claim 8, **characterised in that**, in step b), a substrate containing nitrates, ammonium salts, yeast extract, soybean meal, cottonseed meal, casein, casein hydrolysate, wheat gluten and maize steep-water is used.

10. The process according to one of claims 1 to 9, **characterised in that**, in step b), a substrate containing sulfates, nitrates, chlorides, carbonates and phosphates of the metals sodium, potassium, magnesium, manganese, calcium, zinc and iron or mixtures of two or more of these compounds is used.

11. The process according to one of claims 1 to 10, **characterised in that** step b) is carried out at a temperature in the range of 10 to 40°C.

12. The process according to one of claims 1 to 11, **characterised in that** step b) is carried out at a pH value in the range of 4 to 11.

13. *Yarrowia lipolytica* HR 145 (DSM 12397).

14. The use of *Yarrowia lipolytica* HR 145 (DSM 12397) for the production of γ-decalactone.

## Revendications

1. Procédé pour la préparation de la γ-décalactone, comprenant les stades opératoires suivants :
a) sélection d'une souche de *Yarrowia lipolytica* capable de produire plus de 11 g/l de γ-décalactone en moins de 70 heures, et
b) dans un substrat, culture de la souche sélectionnée en a) pour préparation de la γ-décalactone.

2. Procédé selon revendication 1, **caractérisé en ce que**, au stade b), on cultive une culture pure de la souche sélectionnée au stade a).

3. Procédé selon l'une des revendications 1 à 2, **caractérisé en ce que**, au stade a), on sélectionne *Yarrowia lipolytica* HR 145 (DSM 12397).

4. Procédé selon une des revendications 1 à 3, **caractérisé en ce que**, pour la culture au stade b), on utilise un substrat synthétique, semi-synthétique ou complexe.

5. Procédé selon une des revendications 1 à 4, **caractérisé en ce que**, pour la culture au stade b), on utilise des substrats contenant des composés carbonés et azotés, des sels minéraux, des oligo-éléments et/ou des vitamines.

6. Procédé selon revendication 5, **caractérisé en ce que** l'on utilise en tant que composés carbonés des sucres, les alcools des sucres, un alcool, des acides organiques, des mélanges complexes, des huiles ou des mélanges de deux ou plusieurs de ces substances.

7. Procédé selon revendication 6, **caractérisé en ce que**, au stade b), on utilise un substrat contenant du glucose, du glycérol, du mannitol, de l'acide citrique, de l'extrait de malt, de l'extrait de levure, de la caséine, un hydrolysat de caséine et de l'huile de ricin ou un mélange de deux ou plusieurs de ces substances.

8. Procédé selon une des revendications 1 à 7, **caractérisé en ce que**, au stade b), on utilise un substrat contenant des composés inorganiques et/ou des composés organiques.

9. Procédé selon revendication 8, **caractérisé en ce que**, au stade b), on utilise un substrat contenant des nitrates, des sels d'ammonium, de l'extrait de levure, de la farine de soja, de la farine de graine de coton, de la caséine, un hydrolysat de caséine, du gluten de blé et des liqueurs de macération de maïs.

10. Procédé selon une des revendications 1 à 9, **caractérisé en ce que**, au stade b), on utilise un substrat contenant des sulfates, des nitrates, des chlorures, des carbonates et des phosphates des métaux sodium, potassium, magnésium, manganèse, calcium, zinc et fer ou des mélanges de deux ou plusieurs de ces composés.

11. Procédé selon l'une des revendications 1 à 10, **caractérisé en ce que**, au stade b), on opère à une température dans l'intervalle de 10 à 40°C.

12. Procédé selon l'une des revendications 1 à 11, **caractérisé en ce que**, au stade b), on opère à un pH dans l'intervalle de 4 à 11.

13. *Yarrowia lipolytica* HR 145 (DSM 12397).

14. Utilisation de *Yarrowia lipolytica* HR 145 (DSM 12397) pour la préparation de la γ-décalactone.
